Europäisches Patentamt

European Patent Office

Office européen des brevets

(19)

(11) Numéro de publication: **0 043 326**

**A2**

(12) # DEMANDE DE BREVET EUROPEEN

(21) Numéro de dépôt: **81401042.7**

(22) Date de dépôt: **29.06.81**

(51) Int. Cl.³: **C 07 F 13/00**
C 07 C 57/12, C 07 C 61/29
C 07 C 51/41, C 07 C 143/34
C 07 F 9/38, C 09 F 9/00
C 10 L 1/14, C 10 L 1/18
C 10 L 1/24, C 10 L 1/26

(30) Priorité: **01.07.80 FR 8014650**

(43) Date de publication de la demande:
**06.01.82 Bulletin 82/1**

(84) Etats contractants désignés:
**AT BE CH DE GB IT LI LU NL SE**

(71) Demandeur: **GAMLEN EUROPE S.A.**
**2, rue Huntziger**
**F-92112 Clichy(FR)**

(72) Inventeur: **Brisset, Guy**
**9, place général de Gaulle**
**F-28200 Vernon(FR)**

(72) Inventeur: **Mas, Michel 7, Rue Jean-François Millet**
**Résidence le Val Fourré**
**F-78200 Mantes la Jolie(FR)**

(74) Mandataire: **Weinstein, Zinovi et al,**
**Cabinet Z. WEINSTEIN 20, Avenue de Friedland**
**F-75008 Paris(FR)**

(54) **Sels organosolubles de manganèse, procédé de préparation de ceux-ci, compositions organiques, combustibles liquides, peintures et vernis contenant lesdits sels.**

(57) La présente invention concerne de nouveaux composés organosolubles du manganèse.

Selon l'invention, ces composés sont des sels complexes organométalliques d'acide organique ou organo-métalloïdique dans lesquels le rapport $R$ du nombre d'équivalents d'acide au nombre d'atomes de manganèse est inférieur à 2. On les prépare en faisant réagir à chaud, en milieu organique ou hydrooranique, l'acide organique et un complexe ammine manganeux, prépaé *in-situ* dans le milieu organique réactionnel.

Les composés selon l'invention sont utiles en tant qu'agents siccatifs pour peintures et vernis et en tant qu'adjuvants de combustion des combustibles liquides.

EP 0 043 326 A2

Sels organosolubles de manganèse, procédé de préparation de ceux-ci, compositions organiques, combustibles
liquides peintures et vernis contenant lesdits sels

La présente invention concerne des sels organosolubles de manganèse, un procédé de préparation de ceux-
ci, des compositions organiques, combustibles liquides,
peintures et vernis contenant lesdits sels organosolubles de manganèse.

Les sels organosolubles de manganèse sont utilisés comme catalyseurs d'oxydation dans les opérations chimiques, et notamment comme adjuvant de combustion pour
les combustibles liquides des générateurs thermiques
tels que brûleurs à fuel, moteurs diesel ou propulseurs
à réaction etc, et comme adjuvant de siccativité dans
l'industrie des peintures et des vernis.

Selon l'art antérieur, les composés organosolubles de
manganèse sont préparés par réaction d'un agent chélatant en milieu organique, tel que des dérivés aminés,
des dérivés borés, des dérivés $\beta$-dicétoniques, des
dérivés cyclopentadiéniques, sur un sel hydrosoluble
de manganèse.

Un autre procédé connu consiste à faire réagir un sel
alcalin d'acide organosoluble sur un sel hydrosoluble
de manganèse.

Ces réactions sont sensiblement stoechiométriques,
c'est-à-dire que les composés obtenus ont une composition dans laquelle un atome de manganèse divalent est
lié sensiblement à deux chaines organiques.

Les composés organosolubles de manganèse, ainsi obtenus, sont d'un coût élevé, notamment ceux issus des

dérivés $\beta$ -dicétoniques et cyclopentadiéniques.

D'autre part, un nombre relativement important de molécules organiques par rapport au nombre d'atomes de manganèse est nécessaire dans la mise en oeuvre des procédés de l'art antérieur indiqué ci-dessus, pour obtenir un composé organosoluble de manganèse utilisable comme catalyseur d'oxydation dans différentes opérations chimiques.

La présente invention a pour but de remédier à ces inconvénients en fournissant une solution qui permet de disposer de sels organosolubles de manganèse particulièrement efficaces, à un coût relativement faible, présentant un nombre relativement peu important de molécules organiques par rapport au nombre d'atomes de manganèse.

De préférence, cette solution permet également de fournir des sels organosolubles de manganèse présentant une solubilité vraie dans les solvants organiques et huiles, prouvée par l'absence d'effet Tyndall dans les solutions organiques des sels précités.

Cette solution consiste selon la présente invention en des sels organosolubles de manganèse caractérisés en ce qu'ils sont constitués par des sels complexes organo-métalliques d'acides organiques ou organométalloïdiques, dans lesquels le rapport $\underline{R}$ du nombre d'équivalents d'acide au nombre d'atomes de manganèse est inférieur à 2. Par le terme "nombre d'équivalents d'acide", il faut entendre le nombre de molécules d'acide organique lorsque l'acide utilisé est monofonctionnel, et il faut doubler ou tripler ce "nombre d'équivalents d'acide", dans le cas de di, tri-acide, et plus généralement, le multiplier par le nombre de fonctions acides dans le cas de polyacides.

De préférence, le rapport R sera compris entre 0,2 et 2.

Selon une caractéristique de l'invention, l'acide organique ou organométalloïdique possédant au moins une fonction acide, c'est-à-dire mono, di, ou polyacide, est choisi parmi les acides organiques ou or-ganométalloïdiques ayant de 8 à 30 atomes de carbone et préférablement de 8 à 25 atomes de carbone.

Avantageusement, cet acide organique ou organométal-loïdique possède au moins une fonction acide carboxylique ou sulfonique ou sulfurique ou phosphorique, et appartient de préférence aux séries suivantes :

- aliphatique, à chaîne saturée ou non, droite ou ramifiée,

- cyclique, pouvant être alcoyle-substitué par exemple les acides naphténiques,

- aromatique, éventuellement substitué par un radical alcoyle, notamment les acides alcoyl aryl carboxyliques, les acides alcoylaryl sulfoniques et les acides alcoylaryl phosphoriques,

- terpénique, par exemple les acides résiniques.

La présente invention a également pour objet de fournir des compositions organiques, des combustibles liquides, peintures ou vernis, contenant au moins un sel organosoluble de manganèse selon la présente invention.

La concentration en manganèse dans les compositions organiques est relativement élevée et peut atteindre 400g de manganèse par litre de ladite composition organique. Lesdites compositions conservent, néanmoins, une grande fluidité, puisque leur viscosité varie entre 7 et 450 cst.

En outre, lesdites compositions organiques montrent une très grande stabilité, puisque des solutions orniques concentrées desdits sels organosolubles de manganèse ne donnent jamais lieu à une précipitation d'oxyde de manganèse égale ou supérieure à 0,5% du manganèse en solution, même après un an de conservation.

Un autre objet de la présente invention est de prévoir un procédé de fabrication des sels organosolubles de manganèse, selon l'invention, caractérisé en ce qu'on fait réagir en milieu hydroorganique, un complexe ammine manganeux et l'acide organique ou organométalloïdique précité, en utilisant un rapport R, c'est-à-dire le nombre d'équivalents d'acide par rapport au nombre d'atomes de manganèse, inférieur à 2, et de préférence compris entre 0,2 et 2.

Par "complexe ammine manganeux" il faut entendre un complexe de manganèse divalent comprenant des molécules d'ammoniac ou des molécules d'amines.

Avantageusement, le complexe ammine manganeux est préparé, in-situ, par action, dans le milieu hydroorganique contenant l'acide organique précité, d'un excès de base azotée, tel que par exemple, l'ammoniaque, la diéthylamine ou la méthylamine, sur un sel hydrosoluble de manganèse. Le sel hydrosoluble de manganèse est, de préférence, un sel manganeux tel que par exemple le sulfate, le chlorure, l'acétate, le carbonate ou le nitrate manganeux.

La réaction s'effectue à température moyennement élevée, par exemple comprise entre 40 et 60°C, sous agitation. En fin de réaction, pour favoriser la séparation de l'eau résiduelle provenant notamment de l'ammoniaque, et éventuellement de l'eau de cristallisation du sel hydrosoluble de manganèse, de la phase organique, la température du milieu réactionnel est élevée de 20 à 50°C par rapport à la température de réaction, de telle sorte que ladite température en fin de réaction soit préférablement comprise entre 80 et 90°C.

En outre pour obtenir des solutions concentrées en manganèse, il est avantageux d'additionner, le sel hydrosoluble de manganèse et la base azotée, dans la phase organique, en plusieurs étapes successives. De plus, la concentration en manganèse dans les solutions organiques peut être également contrôlée par l'ajustement de la proportion du solvant dans la phase organique.

La phase organique est séparée de l'eau et des particules solides, par décantation et centrifugation.

Les solutions ainsi obtenues après séparation peuvent être ajoutées telles quelles, après ajustement de la concentration, aux combustibles liquides, peintures, vernis précédemment mentionnés, pour servir selon le cas de composition adjuvant de combustion ou de composition siccative.

D'autres buts, caractéristiques ou avantages de la présente invention apparaîtront mieux au cours de la description ci-après, en référence à plusieurs exemples non limitatifs de mise en oeuvre de cette invention.

Exemple 1.

Dans 750 cm$^3$ de white spirit, on dissout 260 g d'acide oléïque technique (indice d'acide = 193) et, sous bonne agitation on disperse 870 g d'acétate de manganèse tétrahydraté. Sous agitation, on verse lentement 1.200 cm$^3$ d'ammoniaque à 20° Baumé. La réaction élève la température du milieu à 45°C environ, en fin d'addition. Le mélange est ensuite maintenu pendant 5 heures à 55°C sous agitation plus énergique. Ensuite le mélange est porté à 85°C, pour assurer la décantation de l'eau. On élimine l'eau résiduelle après avoir laissé reposer la solution, par décantation.

On centrifuge la phase organique surnageante de façon à éliminer les petites quantités d'oxyde et d'eau qui restent en suspension. On obtient ainsi une solution organique de couleur brun foncé, d'un sel complexe de manganèse. Le rapport $\underline{R}$ représentant le nombre de molécules d'acide oléïque sur le nombre d'atomes de manganèse est égal à 0,26. Pour calculer ce rapport $\underline{R}$, on dose le manganèse contenu dans la phase organique.

Cette solution organique est très fluide (viscosité à 20°C = 8,5 cst) et a une concentration égale à 155 g par litre en manganèse.

Après avoir été conservé six mois, la concentration en manganèse dans la solution organique n'a sensiblement pas variée.

Exemple 2.

Dans 600 cm$^3$ de white spirit, on verse, sous agitation énergique 800 g d'acide gras provenant de résidu de l'industrie papetière (acide gras de "Tallöl") ayant un indice d'acide de 182. On disperse, sous agitation, 200 g de sulfate de manganèse monohydraté, dans la solution à laquelle on ajoute lentement 400 ml d'ammoniaque à 20° Baumé. Cette dernière opération est répétée deux autres fois. Le temps de réaction entre chaque addition de sulfate de manganèse et d'ammoniaque est de 2 heures, la température du milieu réactionnel est maintenue entre 50 et 55°C. A la fin du troisième rajout, on laisse tourner 1 heure et on élève progressivement la température à 85°C pour assurer la décantation de l'eau à la partie inférieure. Après séparation de l'eau, la phase organique obtenue contient 128 g de manganèse par litre.

En suivant le même mode opératoire, on recharge la phase organique avec 100 g de sulfate de manganèse et 1.200 cm$^3$ d'ammoniaque. La nouvelle solution organique obtenue a une concentration de 252 g par litre en manganèse.

Après une troisième charge analogue à la seconde, de sulfate de manganèse et d'ammoniaque, la phase aqueuse se situe, à ce moment, à la partie supérieure. La phase organique est centrifugée de façon à éliminer l'eau et les oxydes résiduels.

Le sel complexe de manganèse qu'elle contient montre un rapport R du nombre de molécules d'acide gras au nombre d'atomes de manganèse égal à 0,23.

Cette solution organique est soluble dans les solvants

-8-

aliphatiques et sa viscosité est de 330 cst à 20°C.

Cet exemple illustre que par trois charges successives de sel hydrosoluble de manganèse et d'ammoniaque, on obtient une solution organique à très forte concentration en manganèse (375 g/l de manganèse).

Exemple 3.

On procède selon le mode opératoire décrit dans l'exemple 1, avec addition de 330 g d'acide linoléïque (indice d'acide 195) dissous dans 200 $cm^3$ de white spirit, de 205 g de carbonate manganeux et de 280 $cm^3$ d'ammoniaque.

La solution organique brune obtenue contient 148 g par litre de manganèse.

Le sel organosoluble de manganèse, contenu dans la présente solution, a un rapport R égal à 0,66.

Exemple 4.

On procède selon le mode opératoire décrit dans l'exemple 2, à la réaction de 460 g d'acide dodécybenzène sulfonique pur dilué dans 500 $cm^3$ de white spirit, 740 g de chlorure manganeux anhydre et 1.100 $cm^3$ d'ammoniaque à 20° Baumé.

L'opération s'effectue en deux recharges.

On obtient une solution organique d'un sel complexe de manganèse dans lequel le rapport R du nombre de molécules de l'acide organique au nombre d'atomes de manganèse est égal à 0,24. La quantité de ce sel complexe contenu dans ladite solution correspond à 245 g de manganèse par litre.

Exemple 5.

On opère comme dans l'exemple 2, à la réaction de 430 g d'acide dinonylphénoxy phosphorique ayant un indice d'acide égal à 240, dissous dans 250 cm$^3$ de white spirit, 398 g de nitrate manganeux hexahydraté et 1.350 cm$^3$ d'ammoniaque.

L'opération s'effectue en deux recharges successives.

La solution brune obtenue contient 102 g par litre de manganèse.

Le sel complexe de manganèse, contenu dans la solution, présente un rapport R entre le nombre de molécules d'acide et le nombre d'atomes de manganèse égal à 0,73.

Bien entendu, la présente invention n'est nullement limitée aux modes d'exécution décrits qui n'ont été donnés qu'à titre d'exemple. En particulier, elle comprend tous les moyens constituant des équivalents techniques des moyens décrits ainsi que leurs combinaisons, si celles-ci sont exécutées suivant son esprit et mises en oeuvre dans le cadre des revendications qui suivent.

Revendications

1. Sel organosoluble de manganèse, soluble dans les milieux organiques, utile notamment comme adjuvant de combustion des combustibles liquides et comme adjuvant de siccativité pour peintures et vernis, caractérisé en ce qu'il est constitué par un sel complexe organométallique d'acide organique ou organométalloïdique dans lequel le rapport R du nombre d'équivalents d'acide au nombre d'atomes de manganèse est inférieur à 2.

2. Sel selon la revendication 1, caractérisé en ce que ledit rapport R est de préférence compris entre 0,2 et 2.

3. Sel selon la revendication 1 ou 2, caractérisé en ce que l'acide organique ou organométalloïdique précité possède au moins une fonction acide, et de 8 à 30 atomes de carbone, préférablement de 8 à 25 atomes de carbone.

4. Sel selon la revendication 3, caractérisé en ce que l'acide organique ou organométalloïdique est choisi parmi les acides possédant un radical organique et au moins une fonction acide carboxylique ou sulfonique ou sulfurique ou phosphorique.

5. Sel selon la revendication 4, caractérisé en ce que le radical organique de l'acide précité est soit :
- un radical aliphatique, à chaîne saturée ou non, droite ou ramifiée,
- un radical cyclique, pouvant être alcoyle substitué,
- un radical aromatique, pouvant posséder un groupement alcoyle de substitution,
- un radical terpénique alcoylé ou non.

6. Sel selon la revendication 5, caractérisé en ce que l'acide précité est choisi dans un groupe constitué par l'acide oléïque, l'acide linoléïque, les acides gras de "Tallöl", les acides résiniques, l'acide dodécybenzène sulfonique, l'acide dinonylphénoxy phosphorique, les acides naphténiques.

7. Procédé de fabrication d'un sel organosoluble de manganèse selon l'une des revendications 1 à 6, caractérisé en ce qu'on fait réagir, en milieu hydroorganique un complexe ammine manganeux et l'acide organique précité, de telle sorte que le rapport $R$ précité soit inférieur à 2, et de préférence compris entre 0,2 et 2.

8. Procédé selon la revendication 7, caractérisé en ce que l'on obtient ledit complexe ammine manganeux, dans ledit milieu hydroorganique contenant l'acide organique précité, par action d'un excès de base azotée sur un sel hydrosoluble de manganèse, préférablement un sel hydrosoluble manganeux.

9. Procédé selon la revendication 8, caractérisé en ce que la base azotée est choisie parmi les composés renfermant la fonction amine ou, et de préférence l'ammoniaque.

10. Procédé selon la revendication 7, caractérisé en ce que le sel hydrosoluble de manganèse est un sel manganeux tel que par exemple le sulfate, le chlorure, l'acétate, le carbonate et le nitrate.

11. Procédé selon l'une quelconque des revendications de 7 à 10, caractérisé en ce qu'en vue d'obtenir des solutions concentrées dudit sel organique ou organométalloïdique du manganèse, on additionne dans le milieu organique, le sel hydrosoluble de manganèse et la base azotée, en plusieurs étapes successives.

12. Procédé selon l'une quelconque des revendications de 7 à 11, caractérisé en ce que le milieu hydroorganique est composé d'un solvant organique, tel que par exemple le "white spirit", servant à solubiliser l'acide organique précité, et de l'eau résiduelle issue notamment de la base azotée, et en ce qu'on maintient, pendant la réaction ledit milieu hydroorganique, à une température moyennement élevée, par exemple comprise entre 40 et 60°C.

13. Procédé selon l'une quelconque des revendications de 7 à 12, caractérisé en ce que dans le but de favoriser la séparation de l'eau du milieu organique, on élève, en fin de réaction, la température du milieu réactionnel de 20 à 50°C par rapport à la température initiale, de telle sorte que ladite température de fin de réaction soit préférablement comprise entre 80 et 90°C.

14. Composition utilisée notamment comme adjuvant de combustion des combustibles liquides, ou comme adjuvant de siccativité pour peintures et vernis, caractérisée en ce qu'elle est constituée par la phase organique obtenue par la mise en oeuvre du procédé selon les revendications de 7 à 13.

15. Composition utilisée notamment comme adjuvant de combustion des combustibles liquides, ou comme adjuvant de siccativité pour peintures et vernis, caractérisée en ce qu'elle contient notamment comme principe actif, au moins un sel organosoluble de manganèse selon l'une des revendications de 1 à 6, avec une concentration pouvant atteindre 400 g par litre en manganèse, pour des viscosités inférieures à 450 cst.

16.  Combustibles liquides, peintures, vernis contenant au moins un sel selon l'une des revendications de 1 à 6.